# EUROPEAN PATENT APPLICATION

(11) **EP 2 415 766 A1**
(43) Date of publication of application: **08.02.2012**
(21) Application number: 10758238.9
(22) Date of filing: 29.03.2010
(51) Int. Cl.: C07D 243/12, C07C 269/06, C07C 271/20, C07C 271/28, C07C 303/36, C07C 303/40, C07C 311/16, A61K 31/551, A61P 1/04, A61P 35/00, A61P 35/02, A61P 43/00, C07B 61/00

(54) **METHOD FOR MANUFACTURING BENZODIAZEPINE DERIVATIVE**

(30) Priority: 31.03.2009 JP 2009087746
(71) Applicant: Zeria Pharmaceutical Co., Ltd., Tokyo 103-8351 (JP)
(72) Inventor: TERAUCHI, Masaru, Tokyo 103-8351 (JP)
(74) Representative: Blodig, Wolfgang
(86) International application number: PCT/JP2010/002237
(87) International publication number: WO 2010/113458

(57) **Abstract**

An industrially advantageous method for producing a 1,5-benzodiazepine compound is provided.

A compound (5) is obtained according to the react ion scheme shown below, and this compound is used as an intermediate.

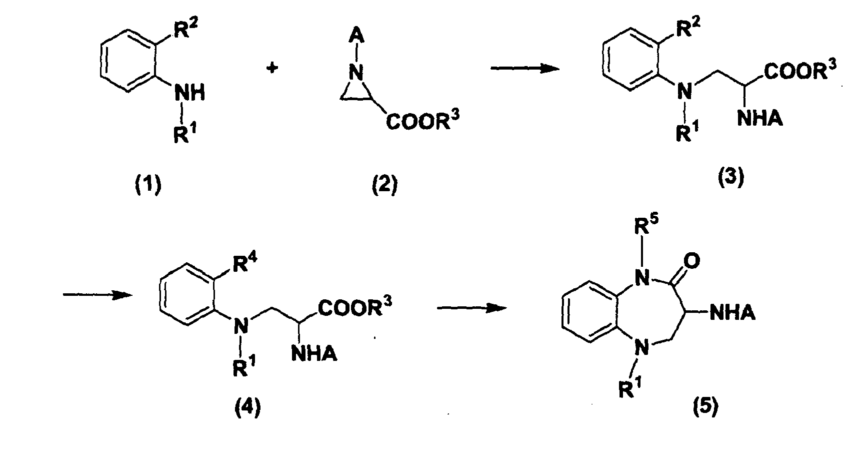

## Description

### [TECHNICAL FIELD]

The present invention relates to an industrially advantageous method for manufacturing a 1,5-benzodiazepine derivative which is useful as a medicine.

### [BACKGROUND ART]

Among 1,5-benzodiazepine compounds, a compound represented by the following formula (A):

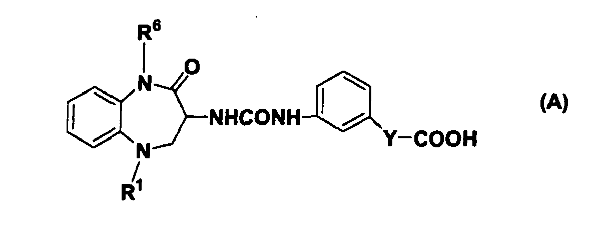

(wherein R¹ represents a linear, branched, or cyclic alkyl group; R⁶ represents an alkyl group or an acylalkyl group; and Y represents a single bond or an alkylidene group) and salts thereof have excellent gastrin/CCK-B receptor antagonist action and excellent gastric acid secretion inhibitory action, and are known to be useful as prophylactic and therapeutic drugs not only for peptic ulcer, but also for gastrointestinal cancer, leukemia, pituitary tumor, small cell lung cancer, thyroid cancer, nervous astrocytoma, cancer pain and the like (Patent Documents 1 to 5).

As a method for producing the compound (A), there is known a method of subjecting 2-nitroaniline to eight steps to obtain a 3-amino-1,5-benzodiazepin-2-one skeleton, and using this skeleton as an intermediate to form desired substituents at the nitrogen atoms of the 1-position and 5-position and at the amino group of the 3-position on the benzodiazepine skeleton, as described in Patent Document 1. However, this method has a large number of steps to obtain the 3-amino-1,5-benzodiazepin-2-one skeleton, and therefore, the method is not suitable as an industrial manufacturing method.

Furthermore, Patent Document 2 describes a method of obtaining 3-amino-2-t-butoxycarbonylaminopropionic acid from aspartic acid as a starting raw material via 2-amino-3-benzyloxycarbonylaminopropionic acid as an intermediate, allowing the obtained product to react with 2-fluoronitrobenzene, further subjecting the reaction product to reduction and ring-closure reactions to form a 3-amino-1,5-benzodiazepine skeleton, and sequentially introducing a cyclohexane skeleton, pinacolic acid and the like into the 3-amino-1,5-benzodiazepine skeleton. However, this method has a large number of steps, and also has a problem that there is a risk of generating hydrogen fluoride, which is a harmful substance, so that the method is not suitable for synthesis in large quantities.

### [Related Art Documents]

### [Patent Documents]

Patent Document 1: WO 1998/025911
Patent Document 2: WO 1999/064403
Patent Document 3: WO 2001/040197
Patent Document 4: WO 2006/077793
Patent Document 5: Japanese Patent Application No. 2008-12873

### [DISCLOSURE OF THE INVENTION]

### [PROBLEMS TO BE SOLVED BY THE INVENTION]

An object of the present invention is to provide an industrially advantageous method for manufacturing a 1,5-benzodiazepine compound of the formula (A), which is useful as a medicine.

### [MEANS FOR SOLVING THE PROBLEMS]

Thus, the inventor of the present invention conducted an investigation on a new method for forming a 3-amino-1,5-benzodiazepin-2-one skeleton, and the inventor found that when an N-alkylaniline derivative to which an alkyl group has been previously introduced into the amino group of the aniline derivative, is allowed to react with N-acylaziridine-2-carboxylic acid, a selective ring-opening reaction at the β-position of aziridine occurs so that N-alkylanilino-2-aminopropionic acid is obtained by a single-step reaction. The inventor also found that when this product is subjected to ring-closure, a 5-alkyl-substituted-3-amino-1,5-benzodiazepin-2-one skeleton is formed with a smaller number of steps and with high yield, and that when this intermediate is involved in the production process, the compound of the formula (A) can be produced in an industrially advantageous manner. Thus, the inventor completed the present invention.

Specifically, the present invention provides a method for producing a compound represented by formula (3) or a salt thereof :

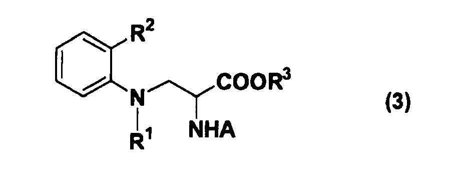

(wherein R¹ represents a linear, branched, or cyclic alkyl group; R² represents a group producing an amino group, an alkylamino group, or an acylalkylamino group through a reduction reaction or a hydrolysis reaction; R³ represents an ester residue; and A represents a protective group containing a sulfonyl group or a carbonyl group),
the method including reacting an aniline derivative represented by formula (1):

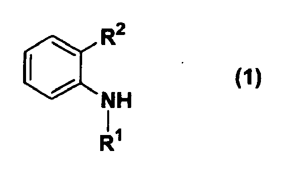

(wherein R¹ and R² respectively have the same meanings as defined above),
with an aziridine derivative represented by formula (2) :

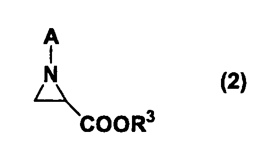

(wherein R³ and A respectively have the same meanings as defined above).

The present invention also provides a method for producing a 1,5-benzodiazepine derivative represented by formula (5):

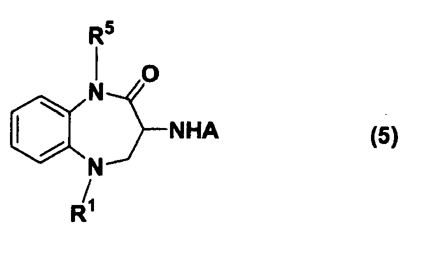

(wherein R¹ represents a linear, branched, or cyclic alkyl group; R⁵ represents a hydrogen atom, an alkyl group or an acylalkyl group; and A represents a protective group containing a sulfonyl group or a carbonyl group),
the method including reacting an aniline derivative represented by formula (1):

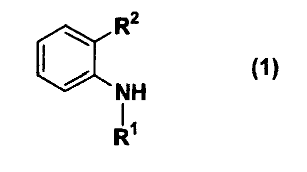

(wherein R² represents a group producing an amino group, an alkylamino group, or an acylalkylamino group through a reduction reaction or a hydrolysis reaction; and R¹ has the same meaning as defined above),
with an aziridine derivative represented by formula (2) :

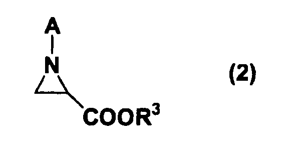

(wherein R³ represents an ester residue; and A has the same meaning as defined above),
to obtain a compound represented by formula (3):

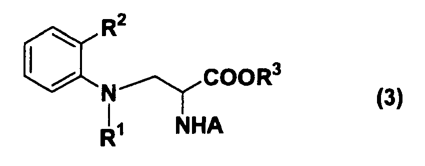

(wherein R¹, R², R³, and A respectively have the same meanings as defined above),
subjecting the compound thus obtained to a reduction reaction or a hydrolysis reaction to obtain a compound represented by formula (4):

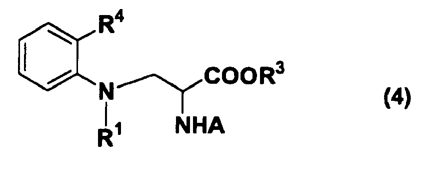

(wherein R⁴ represents an amino group, an alkylamino group, or an acylalkylamino group; and R¹, R³, and A respectively have the same meanings as defined above),
and then subjecting the compound thus obtained to a ring-closure reaction.

The present invention also provides a method for producing a compound represented by formula (A) or a salt thereof:

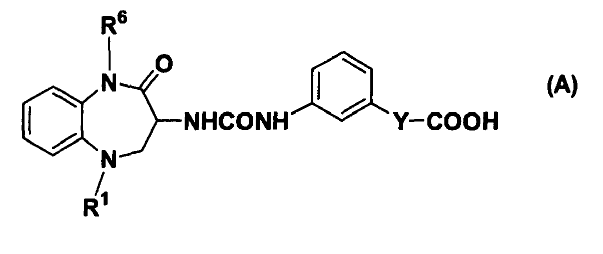

(wherein R¹ represents a linear, branched, or cyclic alkyl group; R⁶ represents an alkyl group or an acylalkyl group; and Y represents a single bond or an alkylidene group),
the method including reacting an aniline derivative represented by formula (1):

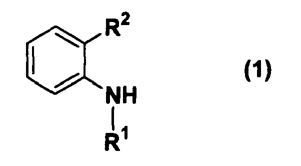

(wherein R² represents a group producing an amino group, an alkylamino group, or an acylalkylamino group through a reduction reaction or a hydrolysis reaction; and R¹ has the same meaning as defined above),
with an aziridine derivative represented by formula (2) :

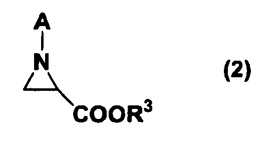

(wherein R³ represents an ester residue; and A represents a protective group containing a sulfonyl group or a carbonyl group),
to obtain a compound represented by formula (3):

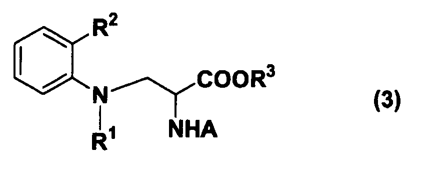

(wherein R¹, R², R³, and A respectively have the same meanings as defined above),
subjecting the compound thus obtained to a reduction reaction or a hydrolysis reaction to obtain a compound represented by formula (4):

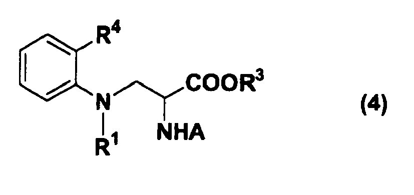

(wherein R⁴ represents an amino group, an alkylamino group, or an acylalkylamino group; and R¹, R³, and A respectively have the same meanings as defined above),
subsequently subjecting the compound thus obtained to a ring-closure reaction to obtain a 1,5-benzodiazepine derivative represented by formula (5):

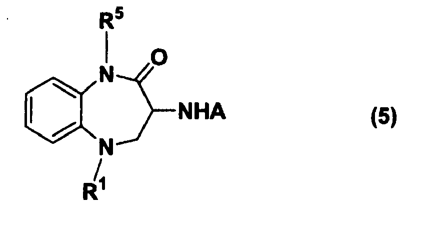

(wherein R⁵ represents a hydrogen atom, an alkyl group, or an acylalkyl group; and R¹ and A respectively have the same meanings as defined above),
allowing the 1,5-benzodiazepine derivative, when R⁵ is a hydrogen atom, to react with an alkyl halide or an acylalkyl halide, subsequently detaching the protective group A to obtain a compound represented by formula (6):

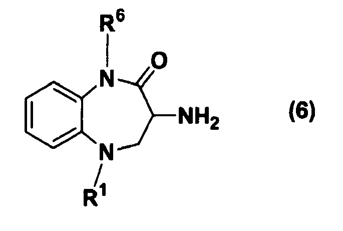

(wherein R¹ and R⁶ respectively have the same meanings as defined above), and
(a) reacting the compound (6) with a compound represented by formula (7):

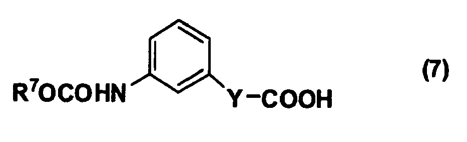

(wherein R⁷ represents an aryl group which may be substituted; and Y has the same meaning as defined above), or (b) reacting the compound (6) with a halogenoformic acid aryl ester, and then reacting the resulting product with a compound represented by formula (8):

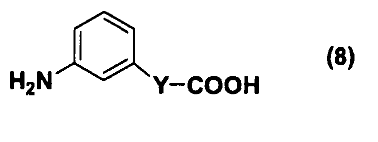

(wherein Y has the same meaning as defined above).

The present invention also provides a compound represented by formula (3a) or a salt thereof:

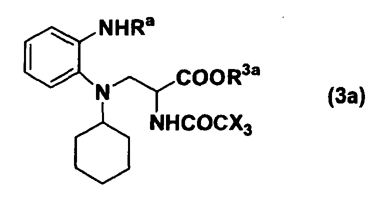

(wherein R^{a} represents a hydrogen atom or a benzyloxycarbonyl group; R^{3a} represents a hydrogen atom or a benzyl group; and X represents a halogen atom).
The present invention also provides a compound represented by formula (5a) or a salt thereof:

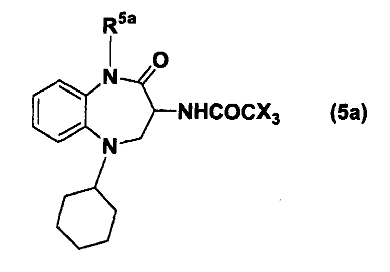

(wherein R^{5a} represents a hydrogen atom or a 3,3-dimethyl-2-oxobutyl group; and X represents a halogen atom).

### [EFFECTS OF THE INVENTION]

According to the method of the present invention, the compound (A) which is useful as a medicine can be produced with a smaller number of steps and with high yield. There is no generation of harmful components suchas hydrogen fluoride during the reaction process, and the method is advantageous for the production in an industrial scale.

### [BEST MODE(S) FOR CARRYING OUT THE INVENTION]

The method of the present invention as represented by a reaction scheme is as follows.

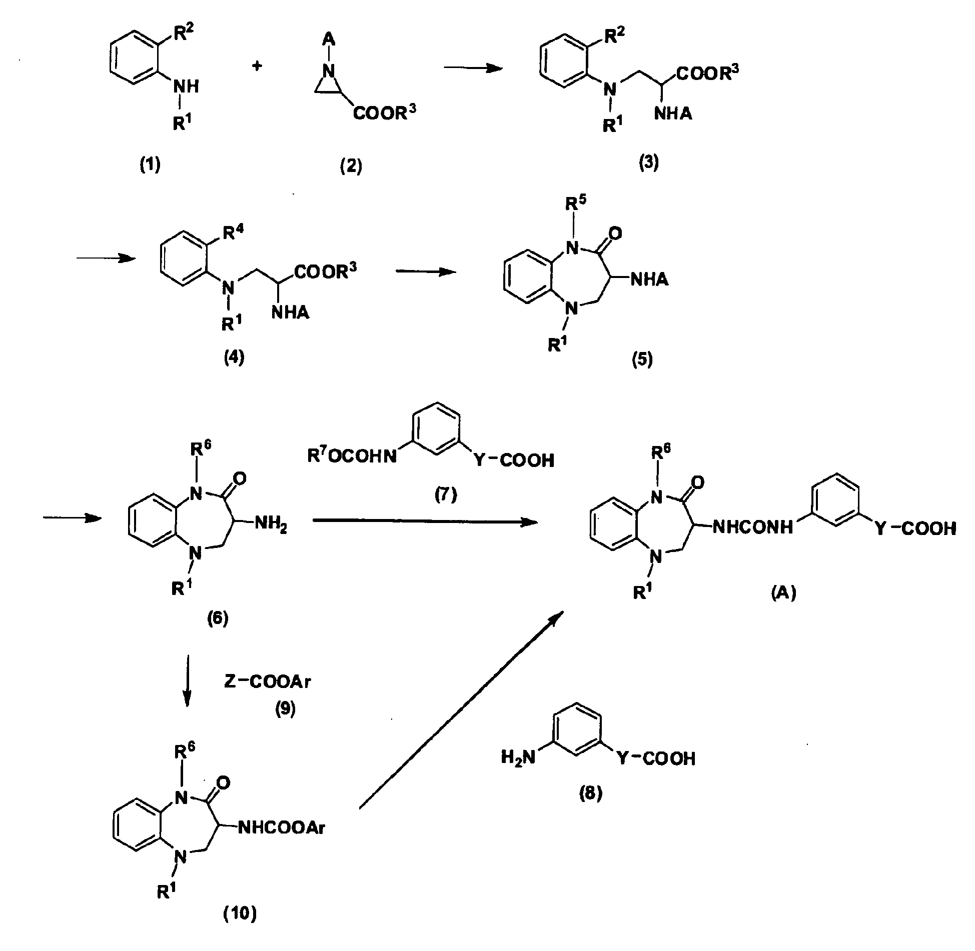

(wherein Z represents a halogen atom; Ar represents an aryl group; and R¹, R², R³, R⁴, R⁵, R⁶, R⁷, A, and Y respectively have the same meanings as defined above.)

The various substituents in the reaction scheme shown above will be explained. R¹ represents a linear, branched, or cyclic alkyl group; however, R¹ is preferably an alkyl group having 1 to 10 carbon atoms, more preferably a linear or branched alkyl group having 1 to 10 carbon atoms, or a monocyclic or bicyclic cycloalkyl group having 3 to 10 carbon atoms, and particularly preferably a cyclohexyl group.

R² represents a group which produces an amino group, an alkylamino group, or an acylalkylamino group through a reduction reaction or a hydrolysis reaction. Among these, examples of the group which produces such an amino group or a substituted amino group through a reduction reaction, include a nitro group, a nitroso group, an azide group, a hydroxyamino group, a benzyloxycarbonylamino group, an N-benzyloxycarbonylalkylamino group, and an N-benzyloxycarbonylacylalkylamino group. Furthermore, examples of the group which produces such an amino group or a substituted amino group through a hydrolysis reaction, include a butoxycarbonylamino group, an N-butoxycarbonylalkylamino group, and an N-butoxycarbonylacylalkylamino group. Among these, a benzyloxycarbonylamino group, an N-benzyloxycarbonylalkylamino group, and an N-benzyloxycarbonylacylalkylamino group are particularly preferred. Here, the alkylamino group may be an alkylamino group having 1 to 6 carbon atoms, and examples thereof include a methylamino group, an ethylamino group, and a propyl amino group. The acylalkylamino group may be a C₂-C₆ alkanoyl-C₁-C₆ alkylamino group, and examples thereof include an acetylmethyl group, a propanoylmethyl group, and a pinacolyl group. Among these, a pinacolyl group is particularly preferred.

Examples of the ester residue represented by R³ include an alkyl group, a benzyl group, a substituted benzyl group, and a trialkylsilyl group. More specific examples include a C₁-C₈ alkyl group, a benzyl group, a halogenobenzyl group, a nitrobenzyl group, and a tri-C₁-C₆ alkylsilyl group.

A represents aprotective group containing a sulfonyl group or a carbonyl group, and examples thereof include a carbamate-based protective group, an amide-based protective group, and a sulfonamide-based protective group, which are conventionally used as protective groups for an amino group. More specific examples include a substituted sulfonyl group such as a p-toluenesulfonyl group or a methanesulfonyl group; an aralkyloxycarbonyl group such as a benzyloxycarbonyl group; an alkyloxycarbonyl group such as a butoxycarbonyl group; an alkanoyl group such as an acetyl group; and a trihalogenoacetyl group such as a trifluoroacetyl group or a trichloroacetyl group. Among these, a trihalogenoacetyl group represented by -COCX₃ (wherein X represents a halogen atom) is particularly preferred because the compound (3) can be selectively obtained, and the detachment reaction of the protective group is achieved with high yield.
Here, X represents a halogen atom, but X is preferably a fluorine atom or a chlorine atom, and particularly preferably a fluorine atom. Z represents a halogen atom, but Z is preferably a chlorine atom or a bromine atom.

Examples of the amino group, alkylamino group, and acylalkylamino group represented by R⁴ include the same groups listed as the examples of R². Also, the same applies to the alkyl group and acylalkyl group represented by R⁵ and R⁶, and examples thereof include an alkyl group having 1 to 6 carbon atoms, and a C₂-C₆ alkanoyl-C₁-C₆ alkyl group.

Examples of the aryl group which may be substituted, as represented by R⁷, and the aryl group represented by Ar include a phenyl group, a halogenophenyl group, an alkylphenyl group, and a nitrophenyl group. Y represents a single bond, or an alkylidene group, and examples thereof include a single bond, a dimethylmethylidene group, and a diethylmethylidene group. However, a single bond is particularly preferred.

Among the compounds of formula (1) as the starting raw material, a compound in which R¹ is a cyclohexyl group and R² is a benzyloxycarbonylamino group, is a novel compound, and can be produced by, for example, allowing 2-cyclohexylaminoaniline to react with benzyloxycarbonyl halide in the presence of a base. On the other hand, among the aziridine compounds of formula (2), N-trifluoroacetylaziridine-2-carboxylic acid benzyl ester is a novel compound, and can be produced by, for example, a method of allowing an aziridine ester to react with trihalogenoacetic anhydride in the presence of a base, according to the descriptions of Bull. Chem. Soc. Jpn., 1978, 51, 1577.

The reaction between a compound (1) and a compound (2) may be carried out in an appropriate solvent at a temperature ranging from room temperature to 200°C, and more preferably, the reaction may be carried out at 80°C to 110°C. The solvent is preferably a non-polar hydrocarbon solvent, and an aromatic hydrocarbon-based solvent such as toluene or xylene is more preferred.

In this reaction between the compound (1) and the compound (2), since a ring-opening reaction at the β-position of aziridine is caused to occur selectively, R¹ is preferably an alkyl group, and particularly preferably a linear alkyl group, a branched alkyl group, or a cycloalkyl group, and more preferably, R¹ is an alkyl group having one hydrogen atom on the carbon atom which is bonded to the nitrogen atom. Therefore, R¹ is preferably a C₁-C₈ linear alkyl group, a C₃-C₈ branched alkyl group, or a C₃-C₆ cycloalkyl group, more preferably a C₃-C₆ cycloalkyl group, and particularly preferably a cyclohexyl group. Furthermore, in order to make the ring-opening reaction at the β-position of aziridine to occur efficiently, the substituent on the nitrogen atom of the aziridine ring is particularly preferably a trihalogenoacetyl group. The substituent A on the nitrogen atom of this aziridine ring is particularly preferably a trifluoroacetyl group, in view of the selectivity of the ring-closure reaction that will be described below.

The ring-opening reaction of aziridine has been conventionally known (WO 97/05129; JP-A-10-203987; and J. Chem. Soc. Perkin Trans. 1, 1997, 3219). However, it has never been known that a ring-opening reaction of aziridine involving an aniline derivative and an N-acylaziridine compound, particularly an N-trihalogenoacetylaziridine compound, such as the reaction of the present invention, occurs in a β-position ring-opening selective manner.

Furthermore, the reaction of the compound (1) and the compound (2) is preferable also from the viewpoint of selectively obtaining a stereoisomer. That is, when a stereoisomer of the compound (2) is used as shown in the following reaction scheme, a stereoisomer of compound (3) may be selectively obtained.

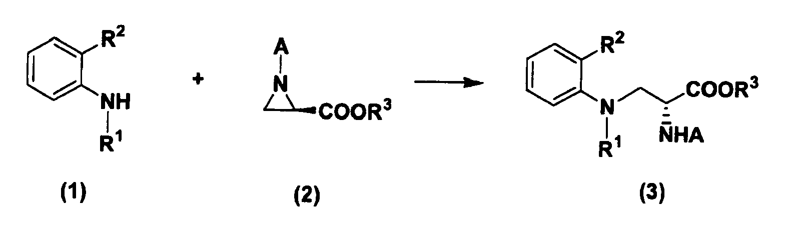

(wherein R¹, R², R³, and A respectively have the same meanings as defined above.)

Therefore, when a stereoisomer of the compound (3) is used, the following stereoisomer of the compound (A) maybe selectively obtained.

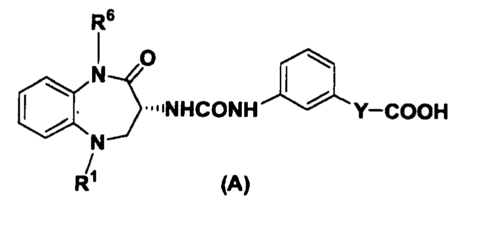

(wherein R¹, R⁶, and Y respectively have the same meanings as defined above.)

When the compound (3) is subj ected to a reduction reaction or a hydrolysis reaction, R³ in the compound (3) is converted to R⁴. During this reaction, the reduction reaction can be carried out, for example, using any of a method of performing a reaction with hydrogen in the presence of a catalyst (catalytic reduction) and a method of using a reducing agent, but catalytic reduction is preferred. The catalyst may be a palladium-based catalyst or a platinum-based catalyst, and examples of the catalyst include palladium-carbon, palladium, platinum oxide, and platinum-carbon. The reaction solvent may be a non-polar hydrocarbon-based solvent, and an aromatic hydrocarbon-based solvent such as toluene or hexane is particularly preferred.

When the compound thus obtained is subjected to a ring-closure reaction, a compound (5) is obtained. This reaction may be carried out at a temperature ranging from room temperature to 80°C in a hydrocarbon-based solvent such as described above.

The reactions starting from the compounds (1) and (2) to obtain the compound (5) can use hydrocarbon-based solvents as the reaction solvent, and can be carried out continuously without isolating a product in the middle of the reactions. Therefore, this process is highly advantageous as an industrial production method.

In the case where R⁵ in the compound (5) is a hydrogen atom, when the compound (5) is allowed to react with an alkyl halide or an acylalkyl halide, R⁵ can be converted to R⁶. This N-alkylation reaction is carried out in the presence of a base such as potassium carbonate, sodium carbonate, or sodium hydroxide, and if necessary, in the presence of a phase transfer catalyst such as tetrabutylammonium bromide. Examples of the reaction solvent that may be used include an ether-based solvent such as tetrahydrofuran or dioxane; toluene, ethyl acetate, N,N-dimethylformamide, and dimethyl sulfoxide. Furthermore, the reaction can also be carried out in a two-phase system such as a water-toluene system, using a phase transfer catalyst such as tetrabutylammonium bromide. The reaction can be carried out usually in a temperature from -78°C to 150°C.

The protective group A of the compound (5) is detached, and thereby a compound (6) can be obtained. The detachment reaction for the protective group A is preferably carried out by detachment through hydrolysis. This reaction may be carried out in the same manner as in conventional hydrolysis reactions, for example, by adding an acid such as hydrochloric acid at 0°C to 100°C. Examples of the solvent that may be used include alcohols such as ethanol; halogenated hydrocarbons such as chloroform; ethers such as dioxane and diethyl ether; and aromatic hydrocarbons such as toluene and xylene.

The reaction starting from the compound (6) to obtain a compound (A) is carried out by any of (a) a method of allowing the compound (6) to react with a compound (7) ; and (b) a method of allowing the compound (6) to react with a halogenoformic acid aryl ester (9) to obtain a compound (10), and then allowing this compound to react with a compound (8) (see WO 2001/040197).

The reaction between the compound (6) and the compound (7) is preferably carried out, for example, in the presence of a base such as triethylamine or potassium carbonate. The reaction is carried out at a temperature in the range of 0°C to the reflux temperature, and examples of the reaction solvent that may be used include dimethylformamide and dimethyl sulfoxide.

The reaction between the compound (6) and the compound (9) is preferably carried out in the presence of a base such as potassium carbonate or triethylamine, and in a solvent such as tetrahydrofuran or dimethylformamide. The reaction temperature is preferably 0°C to the reflux temperature.

The reaction between the compound (10) and the compound (8) is preferably carried out in the presence of a base such as potassium carbonate or triethylamine, and in a solvent such as tetrahydrofuran or dimethylformamide. The reaction is satisfactorily kept at 0°C to room temperature.

The compound (A) thus obtained can be converted to an alkali metal salt, an alkaline earth metal salt or the like by a routine method, but it is preferable to convert the compound into a calcium salt, and it is particularly preferable to add aqueous ammonia and a calcium chloride solution in sequence so as to obtain a calcium salt.

In the reaction scheme shown above, the following compounds (1a), (2a), (3a), and (5a), or salts thereof, are novel and are useful as production intermediates for the compound (A).

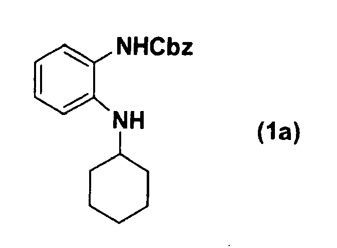

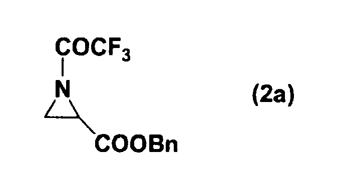

(wherein Cbz represents a benzyloxycarbonyl group; and Bn represents a benzyl group.)

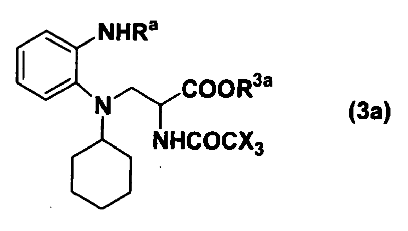

(wherein R^{a} represents a hydrogen atom or a benzyloxycarbonyl group; R^{3a} represents a hydrogen atom or a benzyl group; and X represents a halogen atom.)

Among compounds represented by the compound (3a), a compound in which R^{a} is a hydrogen atom, R^{3a} is a hydrogen atom, and X is a fluorine atom; and a compound in which R^{a} is a benzyloxycarbonyl group, R^{3a} is a benzyl group, and X is a fluorine atom, are particularly preferred.

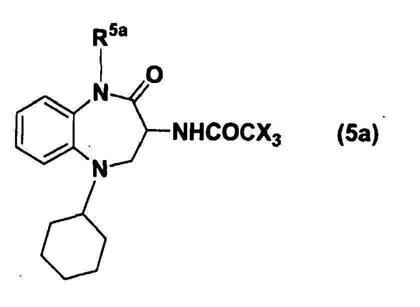

(wherein R^{5a} represents a hydrogen atom, or a 3,3-dimethyl-2-oxobutylgroup; and X represents a halogen atom.)
Among compounds represented by the compound (5a), a compound in which R^{5a} is a hydrogen atom, and X is a fluorine atom; and a compound in which R^{5a} is a 3,3-dimethyl-2-oxobutyl group (a pinacolyl group), and X is a fluorine atom, are particularly preferred.

### [EXAMPLES]

Hereinafter, the present invention will be described in more detail by way of Examples.

### Example 1

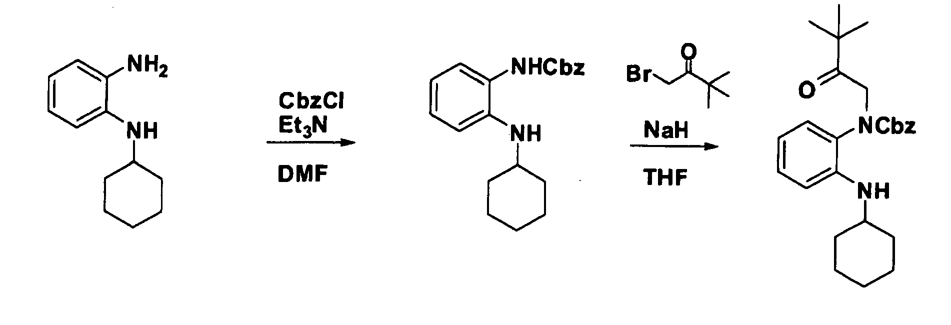

### Production of benzyl 2-(cyclohexylamino)phenyl(3,3-dimethyl-2-oxobutyl)carbamate

### Step 1

### Production of benzyl 2-(cyclohexylamino)phenylcarbamate

5.7 g of N-cyclohexylbenzene-1,2-diamine produced according to a known method (J. Chem. Soc., 1957, 4559) was dissolved in 57 mL of N,N-dimethylformamide, and 3.34 g of triethylamine and 5.63 g of benzyl chlorocarbonate were added to the solution at room temperature. The mixture was stirred for 3 hours at 40°C. 100 mL of water and 100 mL of ethyl acetate were added to the reaction liquid, and the resulting mixture was partitioned. The organic layer was washed sequentially with water and saturated brine, and then was concentrated under reduced pressure. The residue was purified by silica gel column chromatography (n-hexane : ethyl acetate = 10:1 → 5:1), and was further crystallized from IPA/water (5/1). Thus, 8.5 g of the title compound was obtained. Yield: 87%.
¹H-NMR(400MHZ, DMSO-d₆)δ:0.85-1.26 (3H, m), 1.27-1.39 (2H, m), 1.54-1.62 (1H, m), 1.64-1.73 (2H, m), 1.87-1.94 (2H, m), 3.20-3.33 (1H, m), 4.56 (1H, d, J = 6.8 Hz), 5.12 (2H, s), 6.53 (1H, t, J = 7.5 Hz), 6.63 (1H, d, J = 8.2 Hz), 6.96 (1H, t, J = 7.7 Hz), 7.18 (1H, d, J = 5.8 Hz), 7.31-7.46 (5H, m), 8.31 (1H, brs).
MS (FAB) m/z 325 [M+H]⁺

### Step 2

### Production of benzyl 2-(cyclohexylamino)phenyl(3,3-dimethyl-2-oxobutyl)carbamate

In an argon atmosphere, 1.9 g of benzyl 2-(cyclohexylamino)phenylcarbamate was dissolved in 19 mL of tetrahydrofuran, and after the solution is ice-cooled, 279 mg of sodium hydride was added to the solution. The mixture was stirred for 15 minutes. 867 µL of 1-bromo-3,3-dimethyl-2-butanone was slowly added dropwise to the mixture, and the resulting mixture was stirred for another 30 minutes. 100 mL of water and 100 mL of ethyl acetate were added to the reaction liquid, and the resulting mixture was partitioned. The organic layer was washed sequentially with water and saturated brine, and then was concentrated under reduced pressure. The residue was purified by silica gel column chromatography (n-hexane : ethyl acetate = 10:1), and was crystallized from MeOH. Thus, 1.9 g of the title compound was obtained. Yield: 78%.
¹H-NMR(400MHZ, DMSO-d₆)δ:0.99 (1.8H, s), 1.04-1.24 (10.2H, m), 1.24-1.39 (2H, m), 1.53-1.62 (1H, m), 1.64-1.73 (2H, m), 1.81-1.94 (2H, m), 3.21-3.32 (1H, m), 4.07-4.32 (0.8H, m), 4.63-4.72 (1H, m), 4.95-5.12 (1.6H, m), 5.26 (0.2H, d, J = 7.9 Hz), 5.48 (0.4H, d, J = 7.9Hz), 6.47-6.53 (0.8H, m), 6.60-6.66 (1H, m), 6.79 (0.2H, d, J = 4.4Hz), 7.02-7.09 (2H, m), 7.17-7.40 (5H, m).
MS (FAB) m/z 423 [M+H]⁺

### Example 2

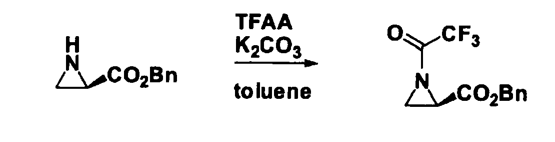

### Production of (R)-benzyl 1-(2,2,2-trifluoroacetyl)aziridine-2-carboxylate

In an argon atmosphere, 1.8 g of (R)-benzyl aziridine-2-carboxylate produced according to a known method (Bull. Chem. Soc. Jpn., 1978, 51, 1577) was dissolved in 18 mL of toluene, and the solution was cooled in a dry ice-acetone bath. 1. 7 g of potassium carbonate and 2.5 g of trifluoroacetic anhydride were added to the solution, and the mixture was directly stirred for 3.5 hours. 18 mL of water was added to the reaction liquid, and the resulting mixture was partitioned. Subsequently, the organic layer was concentrated under reduced pressure. The residue was purified by silica gel column chromatography (n-hexane : ethyl acetate = 10:1), and thus 1.2 g of the title compound was obtained. Yield: 44%.
¹H-NMR(400MHZ, DMSO-d₆)δ: 2.78 (1H, d, J = 3.3 Hz), 2.93 (1H, d, J = 5.9 Hz), 3.87 (1H, dd, J = 3.3, 5.9 Hz), 4.01 (1H, d, J = 7.0 Hz), 4.04 (1H, d, J = 7.0 Hz), 7.36-7.40 (5H, m). MS (FAB) m/z 274 [M+H]⁺

### Example 3

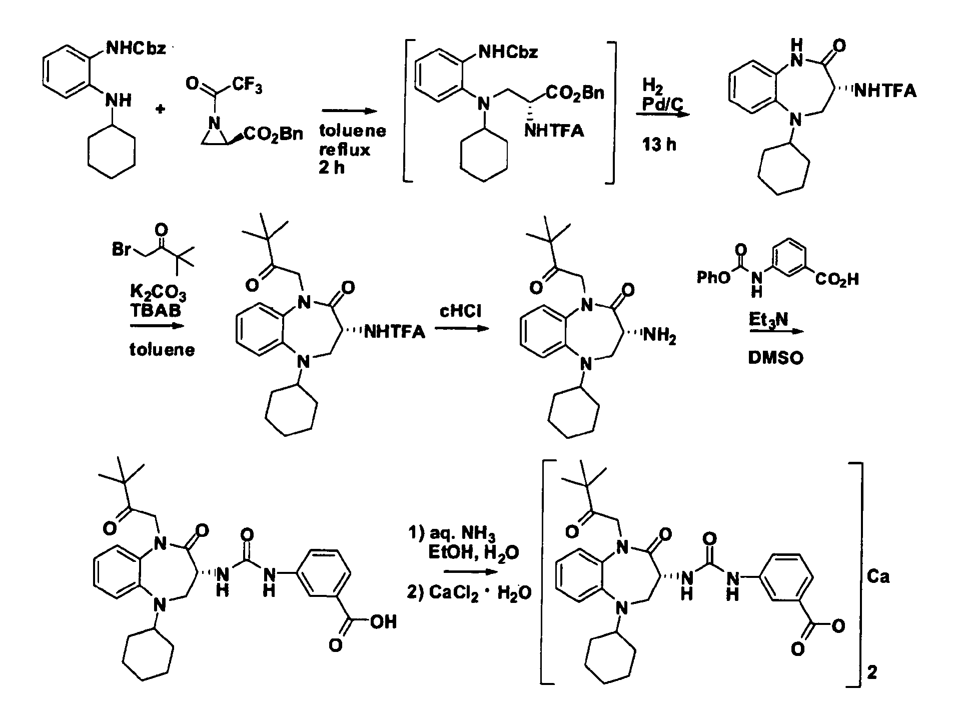

### Production of calcium (R)-3-(3-(5-cyclohexyl-1-(3,3-dimethyl-2-oxobutyl)-2-oxo-2, 3,4,5-tetrahydro-1H-benzo[b][1,4]diazepin-3-yl)ureido)benzo ate

### Step 1

### Production of (R)-N-(1-cyclohexyl-4-oxo-2,3,4,5-tetrahydro-1H-benzo[b][1, 4]diazepin-3-yl)-2,2,2-trifluoroacetamide

324 mg of benzyl 2-(cyclohexylamino)phenylcarbamate obtained according to the method described in Example 1 and 273 mg of (R)-benzyl 1-(2,2,2-trifluoroacetyl)aziridine-2-carboxylate obtained according to the method described in Example 2 were dissolved in 1.45 mL of toluene, and the solution was heated to reflux for 2 hours. Thus, a toluene solution of (R)-benzyl 3-((2-(benzoyloxycarbonylamino)phenyl)(cyclohexyl)amino)-2-(2,2,2-trifluoroacetamide)propionate was obtained.
Furthermore, a portion of the reaction liquid was taken and purified by column chromatography (n-hexane : ethyl acetate = 10:1). Thus, it was confirmed that the product was (R) -benzyl 3-((2-(benzoyloxycarbonylamino)phenyl)(cyclohexyl)amino)-2-(2,2,2-trifluoroacetamido)propionate.
¹H-NMR(400MHZ, DMSO-d₆)δ:0.88-1.17 (5H, m), 1.46-1.53 (1H, m), 1.60-1.68 (2H, m), 1.70-1.78 (2H, m), 2.52-2.61 (1H, m), 3.42 (1H, dd, J = 8.9, 13.3 Hz), 3.58 (1H, dd, J = 5.8, 13.3 Hz), 4.09-4.15 (1H, m), 5.02-5.21 (4H, m), 7.01-7.06 (1H, m), 7.12-7.19 (2H, m), 7.22-7.46 (11H, m), 7.91 (1H, d, J=8.0Hz), 9.92 (1H, d, J = 7.6 Hz).
MS (FAB) m/z 598 [M+H]⁺
The toluene solution of (R)-benzyl 3-((2-(benzoyloxycarbonylamino)phenyl)(cyclohexyl)amino)-2-(2,2,2-trifluoroacetamido)propionate was allowed to cool, and 60 mg of 10% palladium carbon (water content 53%) was added thereto. Subsequently, the mixture was stirred for 13 hours at room temperature in a hydrogen atmosphere. The reaction liquid was filtered and concentrated under reduced pressure, and then the residue was purified by column chromatography (n-hexane : ethyl acetate =10:1). Thus, 312 mgof the title compoundwas obtained. Yield: 88%.
¹H-NMR(400MHZ, DMSO-d₆)δ:1.10-1.42 (4H, m), 1.49-1.68 (4H, m), 1.72-1.81 (1H, m), 1.94-2.02 (1H, m), 3.19-3.27 (1H, m), 3.34 (1H, dd, J = 6.9, 10.3 Hz), 3.62 (1H, t, J = 10.3 Hz), 4.31-4.39 (1H, m), 7.00-7.03 (2H, m), 7.11-7.20 (2H, m), 9.69 (1H, d, J = 8.1 Hz), 9.86 (1H, s).
MS (FAB) m/z 356 [M+H]⁺

Furthermore, when the reaction is completed in 2 hours, and the reaction liquid is purified by column chromatography (methanol : chloroform = 1:10),
(R)-3-((2-aminophenyl)(cyclohexyl)amino)-2-(2,2,2-trifluoro acetamido)propionic acid can be obtained.
¹H-NMR (400MHZ, DMSO-d₆)δ:0.97-1.43 (5H, m), 1.47-2.01 (5H, m), 2.63-2.72 (1H, m), 3.36 (1H, dd, J = 8.1, 13.6 Hz), 3.55 (1H, dd, J = 5.0, 13.6 Hz), 4.10-4.18 (1H, m), 6.50-6.58 (1H, m), 6.68 (1H, dd, J = 1.4, 7.8 Hz), 6.81-6.86 (1H, m), 7.00 (1H, d, J = 7.8 Hz), 9.56 (1H, brs).
MS (FAB) m/z 374 [M+H]⁺

### Step 2

### Production of (R)-N-(5-cyclohexyl-1-(3,3-dimethyl-2-oxobutyl)-2-oxo-2,3,4 ,5-tetrahydro-1H-benzo[b][1,4]diazepin-3-yl)-2,2,2-trifluor oacetamide

200 mg of
(R)-N-(1-cyclohexyl-4-oxo-2,3,4,5-tetrahydro-1H-benzo[b][1, 4]diazepin-3-yl)-2,2,2-trifluoroacetamide was dissolved in 2 mL of toluene, and 94 mg of potassium carbonate, 9 mg of tetrabutylammonium bromide, and 91 µL of
1-bromo-3,3-dimethyl-2-butanone were sequentially added to the solution. The mixture was stirred for 5 hours at room temperature, 10 mL of water was added thereto, and the resulting mixture was partitioned. The organic layer was washed with saturated brine, and then was concentrated under reduced pressure. The residue was purified by silica gel column chromatography (n-hexane : ethyl acetate = 10:1), and thus 155 mg of the title compound was obtained. Yield: 61%.
¹H-NMR(400MHZ, DMSO-d₆)δ:1.10-1.41 (4H, m), 1.15 (9H, s), 1.43-1.65 (3H, m), 1.66-1.83 (2H, m), 1.98-2.16 (1H, m), 3.16-3.27 (2H, m), 3.70 (1H, dd, J = 9.5, 11.6 Hz), 4.44 (1H, dd, J = 7.2, 11.6 Hz), 4.51 (1H, d, J = 8.2Hz), 4.99 (1H, d, J = 8.2 Hz), 7.05-7.14 (2H, m), 7.18-7.30 (2H, m), 9.71 (1H, s).
MS (FAB) m/z 454 [M+H]⁺

### Step 3

### Production of (R)-3-amino-5-cyclohexyl-1-(3,3-dimethyl-2-oxobutyl)-4,5-di hydro-1H-benzo[b][1,4]diazepin-2(3H)-one

155 mg of
(R)-N-(5-cyclohexyl-1-(3,3-dimethyl-2-oxobutyl)-2-oxo-2,3,4 ,5-tetrahydro-1H-benzo[b] [1,4]diazepin-3-yl)-2,2,2-trifluor oacetamide was dissolved in 1.6 mL of toluene, and 1.6 mL of concentrated hydrochloric acid was added thereto. Subsequently, the mixture was stirred for 3 hours at 60°C. 10 mL of water was added to the reaction liquid, and the resulting mixture was partitioned. The organic layer was further extracted with 10 mL of water. The aqueous layer was combined and ice-cooled, and a 25% aqueous solution of sodium hydroxide was added to adjust the aqueous layer to pH 10. The aqueous layer was extracted twice with 10 mL of ethyl acetate, and the ethyl acetate layer thus obtained was washed with 10 mL of saturated brine and then was concentrated under reduced pressure. The residue was purified by silica gel chromatography (chloroform : methanol = 20:1), and thus 119 mg of the title compound was obtained. Yield: 98%.
¹H-NMR(400MHZ, DMSO-d₆)δ:1.11-2.00 (12H, m), 1.17 (9H, s), 3.05-3.23 (3H, m), 3.27-3.32 (1H, m), 4.28 (1H, d, J = 8.0 Hz), 5.10 (1H, d, J = 8.0 Hz), 6.93 (1H, d, J = 7.5 Hz), 7.01 (1H, dd, J = 2.2, 6.3 Hz), 7.02-7.23 (2H, m).
MS (FAB) m/z 358 [M+H]⁺

### Step 4

### Production of (R)-3-(3-(5-cyclohexyl-1-(3,3-dimethyl-2-oxobutyl)-2-oxo-2, 3,4,5-tetrahydro-1H-benzo[b][1,4]diazepin-3-yl)ureido)benzo ic acid monohydrate

740 mg of
(R)-3-amino-5-cyclohexyl-1-(3,3-dimethyl-2-oxobutyl)-4,5-di hydro-1H-benzo[b][1,4]diazepin-2(3H)-one was dissolved in 4 mL of dimethyl sulfoxide, and 810 µL of triethylamine and 532 mg of 3-(phenoxycarbonylamido)benzoic acid were added to the solution. The mixture was stirred for 2 hours at 60°C. Under ice cooling, 7.4 mL of ethanol and 7.4 mL of a 1 N aqueous solution of hydrochloric acid were added thereto, and the resulting mixture was stirred overnight. A solid precipitated therefrom was collected by filtration, and was washed with ethanol/water (1/1). Thus, 780 mg of the title compound was obtained. Yield: 70%.
¹H-NMR(400MHZ, DMSO-d₆)δ:1.13-1.28 (2H, m), 1.18 (9H, s), 1.31-1.42 (2H, m), 1.47-1.70 (4H, m), 1.72-1.80 (1H, m), 1.96-2.02 (1H, m), 3.08-3.46 (3H, m), 4.34-4.42 (2H, m), 5.12 (1H, d, J = 8.1 Hz), 6.61 (1H, d, J = 7.4 Hz), 7.01 (1H, dd, J = 1.0, 7.9 Hz), 7.06-7.12 (1H, m), 7.23-7.34 (3H, m), 7.46-7.50 (2H, m), 7.989(1H, t, J = 1.8Hz), 9.03 (1H, s), 12.92 (1H, brs). MS (FAB) m/z 521 [M+H]⁺

### Step 5

### Production of calcium (R)-3-(3-(5-cyclohexyl-1-(3,3-dimethyl-2-oxobutyl)-2-oxo-2, 3,4,5-tetrahydro-1H-benzo[b] [1,4]diazepin-3-yl)ureido)benzo ate

220 mg of
(R)-3-(3-(5-cyclohexyl-1-(3,3-dimethyl-2-oxobutyl)-2-oxo-2, 3,4,5-tetrahydro-1H-benzo[b][1,4]diazepin-3-yl)ureido)benzo ic acid monohydrate was suspended in 2.2 mL of ethanol, and dissolved by adding 264 µL of concentrated aqueous ammonia under ice cooling. 220 µL of an aqueous solution of 30.5 mg of calcium chloride was added to the reaction liquid, and the mixture was stirred for 30 minutes. Water was added to the mixture, and a powder precipitated therefrom was collected by filtration. The powder collected by filtration was washed with a mixed solvent of water : ethanol = 2:1, and thus 210 mg of the title compound was obtained. Yield: 92%.
¹H-NMR(400MHZ, DMSO-d₆)δ:0.94-1.96 (38H, m), 3.21-3.44 (6H, m), 4.36-4.43 (4H, m), 5.12 (2H, d), 6.77 (2H, d), 7.00-7.29 (10H, m), 7.52-7.56 (4H, m), 7.90 (2H, s), 9.16 (2H, s).
MS (FAB) m/z 1079 [M+H]⁺

### Example 4

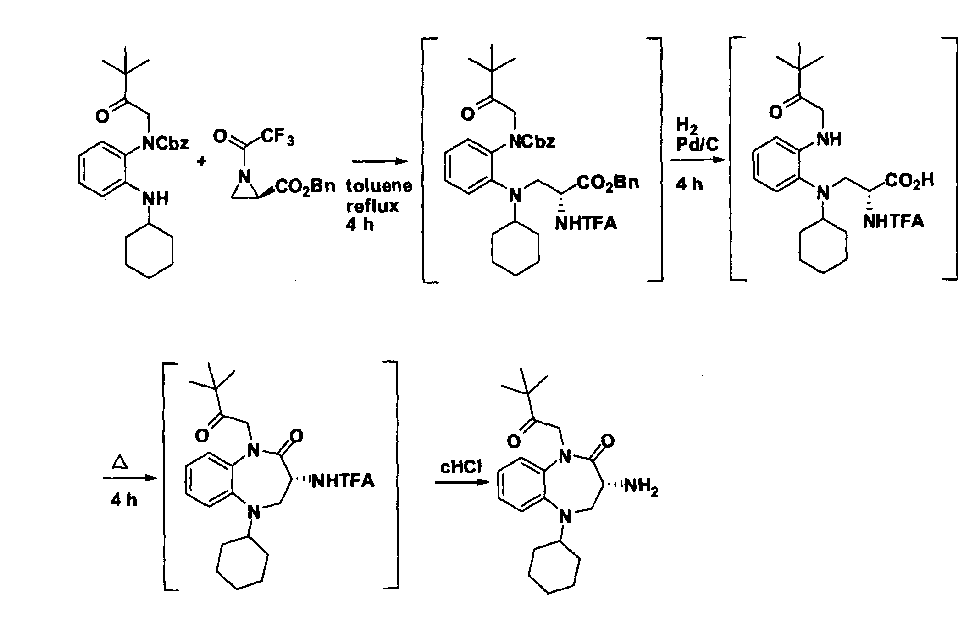

### Production of (R)-3-amino-5-cyclohexyl-1-(3,3-dimethyl-2-oxobutyl)-4,5-di hydro-1H-benzo[b][1,4]diazepin-2(3H)-one

800 mg of benzyl 2-(cyclohexylamino)phenyl (3,3-dimethyl-2-oxobutyl)carbamate obtained according to the method described in Example 1, and 517 mg of (R)-benzyl 1-(2,2,2-trifluoroacetyl)aziridine-2-carboxylate obtained according to the method described in Example 2 were dissolved in 2.74 mL of toluene, and the solution was heated to reflux for 4 hours in an Ar atmosphere. Thus, a toluene solution of (R)-benzyl
3-((2-((benzoyloxycarbonyl) (3,3-dimethyl-2-oxobutyl)amino)p henyl) (cyclohexyl)amino)-2-(2,2,2-trifluoroacetamido)propio nate was obtained.
Furthermore, a portion of the reaction liquid was taken and purified by column chromatography (n-hexane : ethyl acetate = 10:1). Thus, it was confirmed that the product was (R) -benzyl 3-((2-((benzoyloxycarbonyl) (3,3-dimethyl-2-oxobutyl)amino)p henyl) (cyclohexyl)amino)-2-(2,2,2-trifluoroacetamido)propio nate.
¹H-NMR(400MHZ, DMSO-d₆)δ:0.51-1.78 (19H, m), 2.80-3.01 (1H, m), 3.39-3.59 (1H, m), 3.70-3.80 (1H, m), 4.05-4.23 (1H, m), 4.67-5.20 (6H, m), 6.98-7.41 (14H, m), 9.80-9.91 (1H, m). MS (FAB) m/z 696 [M+H]⁺

The toluene solution of (R)-benzyl
3-((2-((benzoyloxycarbonyl) (3,3-dimethyl-2-oxobutyl)amino)p henyl) (cyclohexyl)amino)-2-(2,2,2-trifluoroacetamido)propio nate was left to cool, and 260 mg of 10% palladium-carbon (water content 53%) was added to the solution. Subsequently, the mixture was stirred for 4 hours at room temperature in a hydrogen atmosphere, and thus a toluene solution of
(R)-3-(cyclohexyl-(2-(3,3-dimethyl-2-oxobutylamino)phenyl)a mino)-2-(2,2,2-trifluoroacetamido)propionic acid was obtained.
Furthermore, a portion of the reaction liquid was taken and purified by column chromatography (chloroform : methanol = 10:1). Thus, it was confirmed that the product was (R)-3-(cyclohexyl-(2-(3,3-dimethyl-2-oxobutylamino)phenyl)a mino)-2-(2,2,2-trifluoroacetamido)propionic acid.
¹H-NMR(400MHZ, DMSO-d₆)δ:0.84-1.98 (19H, m), 2.51-2.64 (1H, m), 3.23-4.33 (5H, m), 5.46 (1H, brs), 6.55-6.67 (2H, m), 6.97 (1H, t, J = 7.4 Hz), 7.10 (1H, d, J = 7.8 Hz), 9.39 (1H, d, J = 8.2 Hz), 12-88-13.44 (1H, brs).
MS (FAB) m/z 472 [M+H]⁺

The toluene solution of
(R)-3-(cyclohexyl-(2-(3,3-dimethyl-2-oxobutylamino)phenyl)a mino)-2-(2,2,2-trifluoroacetamido)propionic acid was directly heated to 80°C, and the solution was stirred for 4 hours. After being left to cool, the reaction liquid was filtered, and thus a toluene solution of
(R)-N-(5-cyclohexyl-1-(3,3-dimethyl-2-oxobutyl)-2-oxo-2,3,4 ,5-tetrahydro-1H-benzo[b] [1,4]diazepin-3-yl)-2,2,2-trifluor oacetamide was obtained.
Furthermore, a portion of the reaction liquid was taken and purified by column chromatography (n-hexane : ethyl acetate = 10:1). Thus, it was confirmed that the product was (R)-N-(5-cyclohexyl-1-(3,3-dimethyl-2-oxobutyl)-2-oxo-2,3,4 ,5-tetrahydro-1H-benzo[b][1,4]diazepin-3-yl)-2,2,2-trifluor oacetamide.

2.74 mL of concentrated hydrochloric acid was added to the toluene solution of
(R)-N-(5-cyclohexyl-1-(3,3-dimethyl-2-oxobutyl)-2-oxo-2,3,4 ,5-tetrahydro-1H-benzo[b] [1,4]diazepin-3-yl)-2,2,2-trifluor oacetamide, and the mixture was stirred for 4 hours at 50°C. The mixture was left to cool, subsequently 50 mL of water was added thereto, and the mixture was partitioned. The organic layer was further extracted with 50 mL of water. The aqueous layer was combined and ice-cooled. A 25% aqueous solution of sodium hydroxide was added to adjust the aqueous layer to pH 10. The aqueous layer was extracted twice with 20 mL of ethyl acetate, and the ethyl acetate layer thus obtained was washed with 20 mL of saturated brine and was concentrated under reduced pressure. The residue was purified by silica gel chromatography (chloroform : methanol = 20:1), and thus 419 mg of the title compound was obtained. Yield: 62%.

### Example 5

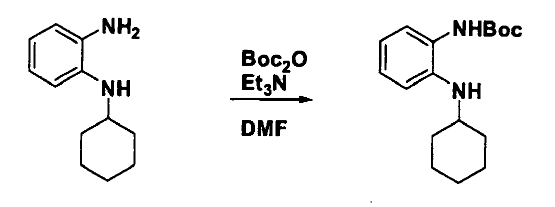

### Production of tert-butyl 2-(cyclohexylamino)phenylcarbamate

Reactions were carried out in the same manner except that di-tert-butyl dicarbonate was used in place of benzyl chlorocarbonate of Example 1, and thus the title compound was obtained. Yield: 86%.
¹H-NMR(400MHZ, DMSO-d₆)δ:1.11-1.25 (3H, m), 1.28-1.40 (2H, m), 1.45 (9H, s), 1.55-1.63 (1H, m), 1.67-1.74 (2H, m), 1.88-1.96 (2H, m), 3.10-3.21 (1H, m), 4.55 (1H, brs), 6.51 (1H, t, J = 7.5 Hz), 6.61 (1H, d, J = 8.2 Hz), 6.91-6.96 (1H, m), 7.13 (1H, d, J = 7.5 Hz), 8.35 (1H, s).
MS (FAB) m/z 291 [M+H]⁺

### Example 6

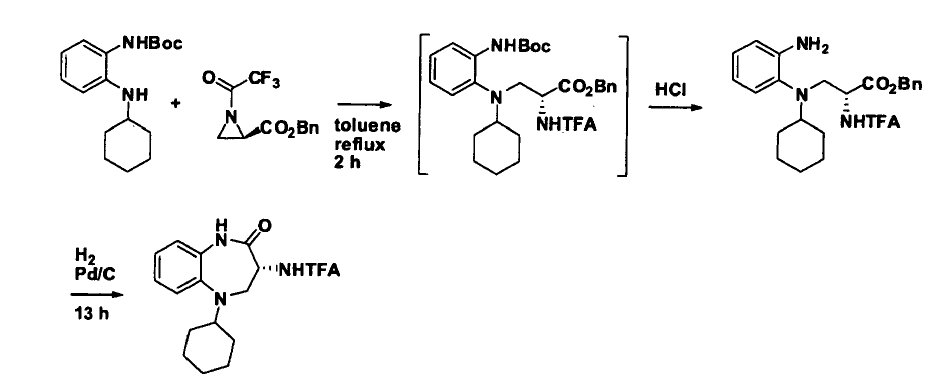

### Production of (R)-N-(1-cyclohexyl-4-oxo-2,3,4,5-tetrahydro-1H-benzo[b][1, 4]diazepin-3-yl)-2,2,2-trifluoroacetamide

### Step 1

### Production of (R)-benzyl 3-((2-aminophenyl)(cyclohexyl)amino)-2-(2,2,2-trifluoroacet amido)propionate

Reactions were carried out in the same manner except that tert-butyl 2- (cyclohexylamino)phenylcarbamate produced by the method described in Example 5 was used in place of benzyl 2-(cyclohexylamino)phenylcarbamate of Example 3. Thus, a toluene solution of (R)-benzyl
3-((2-(tert-butoxycarbonylamino)phenyl)(cyclohexyl)amino)-2 -(2,2,2-trifluoroacetamide)propionate was obtained.
Furthermore, a portion of the reaction liquid was taken and purified by column chromatography (n-hexane : ethyl acetate = 10:1). Thus, it was confirmed that the product was (R) -benzyl 3-((2-(tert-butoxycarbonylamino)phenyl)(cyclohexyl)amino)-2 -(2,2,2-trifluoroacetamido)propionate.
¹H-NMR(400MHZ, DMSO-d₆)δ:0.87 (5H, m), 1.43 (9H, s), 1.47-1.55 (1H, m), 1.63-1.81 (4H, m), 2.51-2.62 (1H, m), 3.42 (1H, dd, J = 9.4, 13.2 Hz), 3.60 (1H, dd, J= 5.4, 13.2 Hz), 4.07-4.14 (1H, m), 5.09 (1H, d, J = 12.7 Hz), 5.17 (1H, d, J = 12.7 Hz), 6.98-7.07 (1H, m), 7.10-7.20 (2H, m), 7.22-7.28 (1H, m), 7.30-7.43 (5H, m), 7.88 (1H, d, J = 8.1 Hz), 9.92 (1H, d, J = 7.8 Hz).
MS (FAB) m/z 564 [M+H]⁺
1. 6 mL of concentrated hydrochloric acid was added to the toluene solution of (R)-benzyl
3-((2-(tert-butoxycarbonylamino)phenyl)(cyclohexyl)amino)-2 -(2,2,2-trifluoroacetamido)propionate, and the mixture was stirred for 4.5 hours at 65°C. After the mixture was left to cool, a saturated aqueous solution of sodium bicarbonate was added to adjust the pH of the reaction liquid to 10, and then the organic layer was separated. The organic layer was washed with saturated brine, and then the residue was purified by column chromatography (n-hexane : ethyl acetate =8:1). Thus, 477 mg of the title compound was obtained. Yield: 56%.
¹H-NMR(400MHZ, DMSO-d₆)δ:0.91-1.24 (5H, m), 1.45-1.90 (5H, m), 2.59-2.70 (1H, m), 3.47-3.71 (2H, m), 4.20-4.39 (1H, m), 4.68-5.12 (3H, m), 7.05-7.40 (9H, m), 9.81-9.90 (1H, brs). MS (FAB) m/z 464 [M+H]⁺

### Step 2

### Production of (R)-N-(1-cyclohexyl-4-oxo-2,3,4,5-tetrahydro-1H-benzo[b] [1, 4]diazepin-3-yl)-2,2,2-trifluoroacetamide

400 mg of (R)-benzyl
3-((2-aminophenyl)(cyclohexyl)amino)-2-(2,2,2-trifluoroacet amido)propionate was dissolved in 4 mL of toluene, and 260 mg of 10% palladium-carbon (water content 53%) was added to the solution. Subsequently, the mixture was stirred for 13 hours at room temperature in a hydrogen atmosphere. The reaction liquid was filtered, and the filtrate was purified by column chromatography (chloroform : methanol = 10:1). Thus, (R)-3-(cyclohexyl-(2-(3,3-dimethyl-2-oxobutylamino)phenyl)a mino)-2-(2,2,2-trifluoroacetamido)propionic acid was obtained.

### Example 7

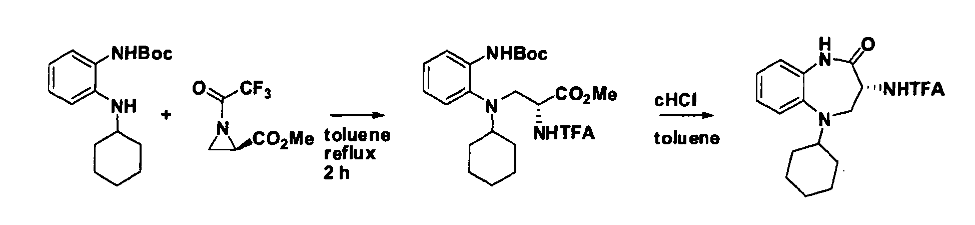

### Production of (R)-N-(1-cyclohexyl-4-oxo-2,3,4,5-tetrahydro-1H-benzo[b][1, 4]diazepin-3-yl)-2,2,2-trifluoroacetamide

### Step 1

### Production of (R)-methyl 3-((2-(tert-butoxycarbonylamino)phenyl)(cyclohexyl)amino)-2 -(2,2,2-trifluoroacetamido)propionate

Reactions were carried out in the same manner except that tert-butyl 2- (cyclohexylamino)phenylcarbamate produced by the method described in Example 5 and (R)-methyl 1-(2,2,2-trifluoroacetyl)aziridine-2-carboxylate produced by a known method (J. Chem. Soc. Chem. Commun., 1987, 153) were used in place of benzyl 2- (cyclohexylamino)phenylcarbamate and (R)-benzyl 1-(2,2,2-trifluoroacetyl)aziridine-2-carboxylate of Example 3. Thus, the title compound was obtained. Yield: 75%.
¹H-NMR(400MHZ, DMSO-d₆)δ:0.91-1.04 (1H, m), 1.06-1.25 (4H, m), 1.44 (9H, s), 1.48-1.56 (1H, m), 1.65-1.82 (4H, m), 2.42-2.63 (1H, m), 3.39 (1H, dd, J = 9.1, 15.2 Hz), 3.56 (1H, dd, J = 5.8, 13.3 Hz), 3.64 (3H, s), 4.09-4.78 (1H, m), 6.98-7.03 (1H, m), 7.13 (1H, t, J = 7.2 Hz), 7.31 (1H, dd, J = 1.1, 7.9 Hz), 7.66 (1H, s), 7.88 (1H, d, J = 7.9 Hz), 9.89 (1H, s).
MS (FAB) m/z 488 [M+H]⁺

### Step 2

### Production of (R)-N-(1-cyclohexyl-4-oxo-2,3,4,5-tetrahydro-1H-benzo[b][1, 4]diazepin-3-yl)-2,2,2-trifluoroacetamide

242 mg of (R)-methyl 3-((2-(tert-butoxycarbonylamino)phenyl)(cyclohexyl)amino)-2 -(2,2,2-trifluoroacetamido)propionate was dissolved in 2.4 mL of toluene, and 484 µL of concentrated hydrochloric acid was added to the solution. The mixture was stirred for one hour at 50°C. The organic layer was separated and was concentrated under reduced pressure. The residue was purified by column chromatography (n-hexane : ethyl acetate = 10:1), and thus 158 mg of the title compound was obtained. Yield: 90%.

### Example 8

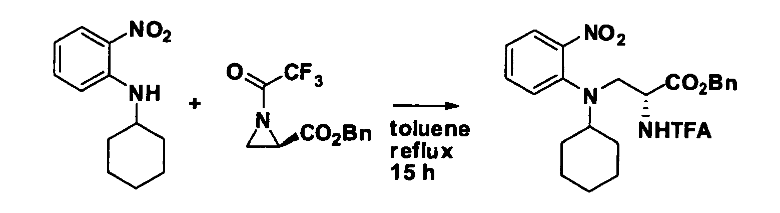

### Production of (R)-benzyl 3-(cyclohexyl-(2-nitrophenyl)amino)-2-(2,2,2-trifluoroaceta mido)propionate

Reactions were carried out in the same manner except that N-cyclohexyl-2-nitroaniline produced by a known method(J.Chem. Soc., 1957, 4559) was used in place of benzyl 2- (cyclohexylamino) phenylcarbamate of Example 3, while the time of heating under reflux was changed to 15 hours. Thus, the title compound was obtained. Yield: 10%.
¹H-NMR(400MHZ, DMSO-d₆)δ:0.96-1.76 (10H, m), 3.06-3.15 (1H, m), 3.53 (1H, dd, J = 8.5, 14.1 Hz), 3.71 (1H, dd, J = 5.6, 14.1 Hz), 4.32-4.42 (1H, m), 5.04 (1H, d, J = 12.7 Hz), 5.13 (1H, d, J = 12.7 Hz), 7.20-7.45 (6H, m), 7.49 (1H, d, J = 7.2 Hz), 7.55-7.60 (1H, m), 7.76 (1H, dd, J = 1.5, 8.1 Hz), 9.58 (1H, d, J = 8.0 Hz).
MS (FAB) m/z 494 [M+H]⁺

### Example 9

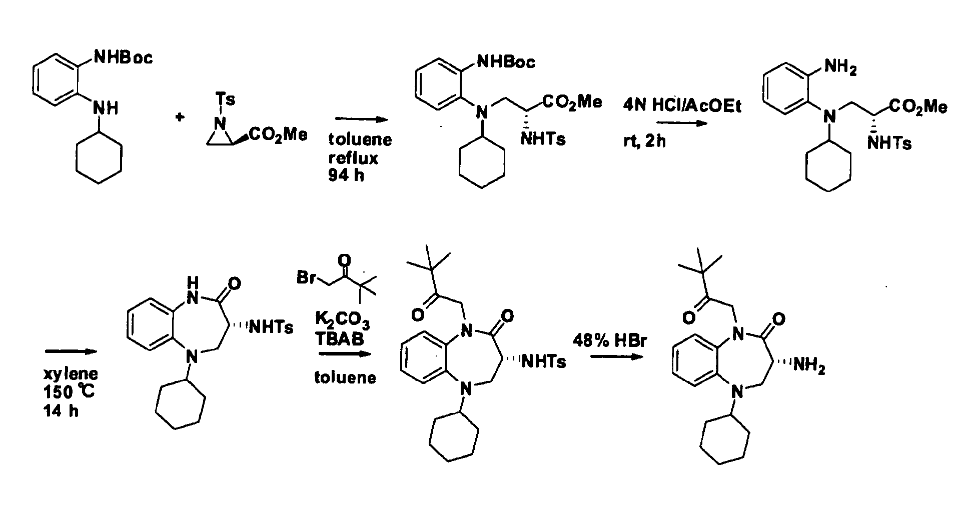

### Production of (R)-3-amino-5-cyclohexyl-1-(3,3-dimethyl-2-oxobutyl)-4,5-di hydro-1H-benzo[b][1,4]diazepin-2(3H)-one

### Step 1

### Production of (R)-methyl 3-((2-(tert-butoxycarbonylamino)phenyl)(cyclohexyl)amino)-2 -(4-methylphenylsulfonamido)propionate

Reactions were carried out in the same manner except that tert-butyl 2- (cyclohexylamino)phenylcarbamate produced by the method described in Example 5 and (R)-methyl 1-tosylaziridine-2-carboxylate produced by a known method (J. Chem. Soc. Chem. Commun., 1987, 153) were used in place of benzyl 2-(cyclohexylamino)phenylcarbamate and (R)-benzyl 1-(2,2,2-trifluoroacetyl)aziridine-2-carboxylate of Example3, while the time of heating under reflux was changed to 94 hours. Thus, the title compound was obtained. Yield: 88%. ¹H-NMR(400MHZ, DMSO-d₆)δ:0.81-1.14 (5H, m), 1.41-1.59 (2H, m), 1.45 (9H, s), 1.60-1.72 (3H, m), 2.49-2.52 (3H, brs), 3.10-3.21 (2H, m), 3.32-3.34 (1H, m), 3.36 (3H, s), 3.40-3.47 (1H, m), 6.89-6.95 (1H, m), 7.01 (1H, d, J = 7.0 Hz), 7.05-7.12 (1H, m), 7.34 (2H, d, J = 7.1 Hz), 7.52 (2H, d, J = 8.3 Hz), 7.79 (1H, d, J= 7.9 Hz), 8.39 (1H, d, J = 8.0 Hz).
MS (FAB) m/z 546 [M+H]⁺

### Step 2

### Production of (R)-methyl 3-((2-aminophenyl)(cyclohexyl)amino)-2-(4-methylphenylsulfo namido)propionate

320 mg of (R)-methyl
3-((2-(tert-butoxycarbonylamino)phenyl)(cyclohexyl)amino)-2 - (4-methylphenylsulfonamido) propionate was dissolved in a 4 N hydrochloric acid-ethyl acetate solution, and the resulting solution was stirred for one hour at room temperature. 10 mL of a saturated aqueous solution of sodium bicarbonate was added to the reaction liquid, and the resulting mixture was partitioned. The aqueous layer was extracted twice with 10 mL of ethyl acetate. The organic layer was combined and was washed with saturated brine. Subsequently, the organic layer was dried over sodium sulfate and was concentrated under reduced pressure. Thus, 270 mg of the title compound was obtained. Yield: 100%. ¹H-NMR(400MHZ, DMSO-d₆)δ:0.89-1.26 (5H, m), 1.46-1.69 (5H, m), 2.36-2.55 (4H, m), 3.07-3.19 (2H, m), 3.29 (3H, s), 3.46-3.53 (1H, m), 4.56 (1H, d, J = 11.0 Hz), 6.44 (1H, t, J = 7.0 Hz), 6.62 (1H, d, J = 7.9 Hz), 6.67-6.72 (1H, m), 6.78 (1H, t, J = 7.3 Hz), 7.35 (2H, d, J = 8.2 Hz), 7.54 ( 2H, d, J = 8.2 Hz). MS (FAB) m/z 446 [M+H]⁺

### Step 3

### Production of (R)-N-(1-cyclohexyl-4-oxo-2,3,4,5-tetrahydro-1H-benzo[b][1, 4]diazepin-3-yl)-4-methylbenzenesulfonamide

268 mg of (R)-methyl
3-((2-aminophenyl)(cyclohexyl)amino)-2-(4-methylphenylsulfo namido)propionate was dissolved in 2.5 mL of o-xylene, and the solution was heated to reflux for 14 hours. The reaction liquid was purified by column chromatography (n-hexane : ethyl acetate = 3:1), and thus 173 mg of the title compound was obtained. Yield: 71%.
¹H-NMR(400MHZ, DMSO-d₆)δ:1.03-1.39 (5H, m), 1.48-1.62 (3H, m), 1.67-1.89 (2H, m), 2.36 (3H, s), 3.09-3.17 (2H, m), 3.28 (1H, t, J = 10.1 Hz), 3.72-3.81 (1H, m), 6.91 (1H, d, J = 7.7 Hz), 6.94-7.00 (1H, m), 7.08-7.16 (2H, m), 7.33 (2H, d, J = 8.2 Hz), 7.57 (2H, d, J = 8.2 Hz), 8.07 (1H, d, J = 6.5 Hz), 9.66 (1H, s).
MS (FAB) m/z 414 [M+H]⁺

### Step 4

### Production of (R)-N-(1-cyclohexyl-4-oxo-2,3,4,5-tetrahydro-1H-benzo[b] [1, 4]diazepin-3-yl)-4-methylbenzenesulfonamide

Reactions were carried out in the same manner except that (R)-N-(1-cyclohexyl-4-oxo-2,3,4,5-tetrahydro-1H-benzo[b][1, 4]diazepin-3-yl)-4-methylbenzenesulfonamide was used in place of
(R)-N-(1-cyclohexyl-4-oxo-2,3,4,5-tetrahydro-1H-benzo[b][1, 4]diazepin-3-yl)-2,2,2-trifluoroacetamide of Example 3, and thus the title compound was obtained. Yield: 88%. ¹H-NMR(400MHZ, DMSO-d₆)δ:1.07-1.40 (5H, m), 1.11 (9H, s), 1.47-1.63 (3H, m), 1.67-1.75 (1H, m), 1.88-1.94 (1H, m), 2.36 (3H, s), 3.04 (1H, dd, J = 7.1, 9.3 Hz), 3.08-3.17 (1H, m), 3.28-3.35 (1H, m), 3.88-3.96 (1H, m), 4.17 (1H, d, J = 18.0Hz), 4. 96 (1H, d, J = 18.0 Hz), 7.02 (1H, d, J = 7.6 Hz), 7.08-7.13 (1H, m), 7.20-7.24 (2H, m), 7.29 (2H, d, J = 8.2Hz), 7.50 (2H, d, J = 8.2 Hz), 8.10 (1H, d, J = 8.7 Hz).
MS (FAB) m/z 512 [M+H]⁺

### Step 5

### Production of (R)-3-amino-5-cyclohexyl-1-(3,3-dimethyl-2-oxobutyl)-4,5-di hydro-1H-benzo[b][1,4]diazepin-2(3H)-one

100 mg of
(R)-N-(1-cyclohexyl-4-oxo-2,3,4,5-tetrahydro-1H-benzo[b][1, 4)diazepin-3-yl)-4-methylbenzenesulfonamide was dissolved in 48% hydrobromic acid, and the solution was heated to reflux for one hour. The reaction liquid was left to cool, and then ethyl acetate and a saturated aqueous solution of sodium bicarbonate were added thereto. The resulting mixture was partitioned. The organic layer was washed with saturated brine, and then the organic layer was dried over sodium sulfate and was concentrated under reduced pressure. The residue was purified by column chromatography (n-hexane : ethyl acetate = 7:1 → 3:1), and thus 25 mg of the title compound was obtained. Yield: 36%.

### Example 10

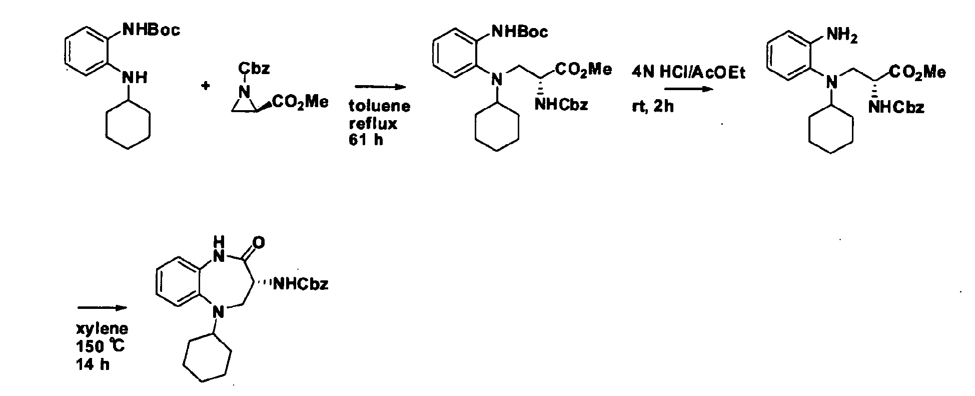

### Production of (R)-methyl 3-((2-aminophenyl)(cyclohexyl)amino)-2-(benzoyloxycarbonyla mino)propionate

### Step 1

### Production of (R)-methyl 3-((2-(tert-butoxycarbonylamino)phenyl)(cyclohexyl)amino)-2 -(4-methylphenylsulfonamido)propionate

Reactions were carried out in the same manner except that tert-butyl 2- (cyclohexylamino)phenylcarbamate produced by the method described in Example 5 and (R)-1-benzyl 2-methylaziridine-1,2-dicarboxylate produced by a known method (J. Chem. Soc. Chem. Commun., 1987, 153) were used in place of benzyl 2-(cyclohexylamino)phenylcarbamate and (R)-benzyl 1-(2,2,2-trifluoroacetyl)aziridine-2-carboxylate of Example3, while the time of heating under reflux was changed to 61 hours. Thus, the title compound was obtained. Yield: 40%.
¹H-NMR(400MHZ, DMSO-d₆)δ:0.90-1.21 (5H, m), 1.44 (9H, s), 1.45-1.55 (1H, m), 1.63-1.82 (4H, m), 3.24-3.39 (6H, m), 3.75-3.83 (1H, m), 4.97 (1H, d, J = 12.5 Hz), 5.03 (1H, d, J = 12.5Hz), 6.91-7.02 (1H, m), 7.09-7.13 (1H, m), 7.25-7.40 (6H, m), 7.74-7.88 (3H, m).
MS (FAB) m/z 526 [M+H]⁺

### Step 2

### Production of (R)-methyl 2-(benzoyloxycarbonylamino)-3-((2-(tert-butoxycarbonylamino )phenyl)(cyclohexyl)amino)propionate

Reactions were carried out in the same manner except that (R)-methyl
3-((2-(tert-butoxycarbonylamino)phenyl)(cyclohexyl)amino)-2 -(4-methylphenylsulfonamido)propionate was used in place of (R)-methyl
3-((2-(tert-butoxycarbonylamino)phenyl)(cyclohexyl)amino)-2 - (4-methylphenylsulfonamido)propionate of Example 9, and thus the title compound was obtained. Yield: 99%.
¹H-NMR(400MHZ, DMSO-d₆)δ:0.92-1.25 (5H, m), 1.48-1.55 (1H, m), 1.63-1.78 (4H, m), 3.20-3.40 (2H, m), 3.50 (3H, s), 3.83-3.92 (1H, m), 4.67-4.77 (3H, m), 4.99 (2H, s), 6.52 (1H, t, J = 7.2 Hz), 6.66 (1H, dd, J = 1.3, 8.0 Hz), 6.79-6.83 (1H, m), 7.00 (1H, d, J = 7.7 Hz), 7.32-7.43 (6H, m).
MS (FAB) m/z 426 [M+H]⁺

### Step 3

### Production of (R)-benzyl 1-cyclohexyl-4-oxo-2,3,4,5-tetrahydro-1H-benzo[b][1,4]diaze pin-3-ylcarbamate

Reactions were carried out in the same manner except that (R)-methyl
2-(benzoyloxycarbonylamino)-3-((2-(tert-butoxycarbonylamino )phenyl)(cyclohexyl)amino)propionate was used in place of (R)-methyl
3-((2-aminophenyl)(cyclohexyl)amino)-2-(4-methylphenylsulfo namido)propionate of Example 9, and thus the title compound was obtained. Yield: 100%.
¹H-NMR(400MHZ, DMSO-d₆)δ:1.09-1.42 (4H, m), 1.44-1.66 (4H, m), 1.72-1.81 (1H, m), 1.92-2.00 (1H, m), 3.16-3.24 (1H, m), 3.26-3.34 (1H, m), 3.43 (1H, t, J = 11.4 Hz), 4.04-4.13 (1H, m), 4.99 (2H, d, J = 1.4 Hz), 6.95-7.01 (2H, m), 7.09-7.20 (2H, m), 7.28-7.38 (5H, m), 7.52 (1H, d, J = 8.7 Hz), 9.68 (1H, s). MS (FAB) m/z 394 [M+H]⁺

### Example 11

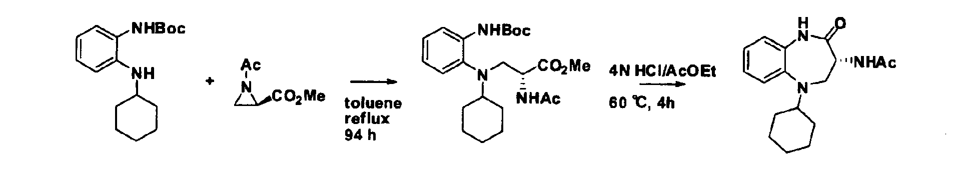

### Production of (R)-N-(1-cyclohexyl-4-oxo-2,3,4,5-tetrahydro-1H-benzo[b][1, 4]diazepin-3-yl)acetamide

### Step 1

### Production of (R)-methyl 2-acetamido-3-((2-(tert-butoxycarbonyl)phenyl) (cyclohexyl)a mino)propionate

Reactions were carried out in the same manner except that tert-butyl 2-(cyclohexylamino)phenylcarbamate produced according to the method described in Example 5 and (R)-methyl 1-acetylaziridine-2-carboxylate produced by a known method (TetrahedronAsymmetry, 1993, 4, 903) were used in place of benzyl 2-(cyclohexylamino)phenylcarbamate and (R)-benzyl 1-(2,2,2-trifluoroacetyl)aziridine-2-carboxylate of Example 3, while the time of heating under reflux was changed to 94 hours. Thus, the title compound was obtained. Yield: 40%. ¹H-NMR(400MHZ, DMSO-d₆) δ:0.79-1.31 (5H, m), 1.46 (9H, s), 1.48-1.56 (1H, m), 1.65-1.81 (4H, m), 1.82 (3H, s), 3.20-3.27 (1H, m), 3.30-3.41 (2H, m), 3.59 (3H, s), 3.92-3.99 (1H, m), 6.97-7.02 (1H, m), 7.09-7.15 (1H, m), 7.26-7.30 (1H, m), 7.76 (1H, brs), 7.83-7.78 (1H, m), 8.31 (1H, d, J = 7.6 Hz).
MS (FAB) m/z 434 [M+H]⁺

### Step 2

### Production of (R)-N-(1-cyclohexyl-4-oxo-2,3,4,5-tetrahydro-1H-benzo[b][1, 4]diazepin-3-yl)acetamide

434 mg of (R)-methyl
2-acetamido-3-((2-(tert-butoxycarbonyl)phenyl)(cyclohexyl)a mino) propionate was dissolved in a 4 N hydrochloric acid-ethyl acetate solution, and the solution was stirred for 4 hours at 60°C. 10mL of a saturated aqueous solution of sodium bicarbonate was added to the reaction liquid, and the resulting mixture was partitioned. The aqueous layer was extracted twice with 10 mL of ethyl acetate. The organic layer was combined, and was washed with saturated brine. Subsequently, the organic layer was dried over sodium sulfate and was concentrated under reduced pressure. Thus, 207 mg of the title compound was obtained. Yield: 62% ¹H-NMR(400MHZ, DMSO-d₆) δ:1.09-1.42 (4H, m), 1.43-1.69 (4H, m), 1.72-1.81 (1H, m), 1.82 (3H, s), 1.91-1.97 (1H, m), 3.15-3.22 (1H, m), 3.25-3.40 (2H, m), 4.36 (1H, dd, J=3.7, 7.8 Hz), 6.98 (2H, d, J = 4.0 Hz), 7.08-7.18 (2H, m), 8.14 (1H, d, J = 8.2 Hz), 9.68 (1H, s).
MS (FAB) m/z 302 [M+H]⁺

## Claims

1. A method for producing a compound represented by formula (3) or a salt thereof: 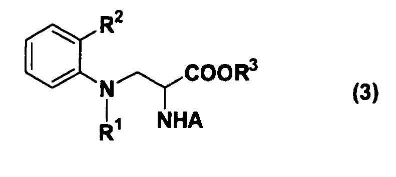 (wherein R¹ represents a linear, branched, or cyclic alkyl group; R² represents a group producing an amino group, an alkylamino group, or an acylalkylamino group through a reduction reaction or a hydrolysis reaction; R³ represents an ester residue; and A represents a protective group containing a sulfonyl group or a carbonyl group),
the method comprising reacting an aniline derivative represented by formula (1): 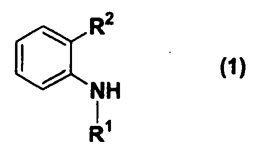 (wherein R¹ and R² respectively have the same meanings as defined above),
with an aziridine derivative represented by formula (2) : 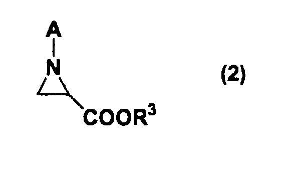 (wherein R³ and A respectively have the same meanings as defined above).

2. A method for producing a 1,5-benzodiazepine derivative represented by formula (5): 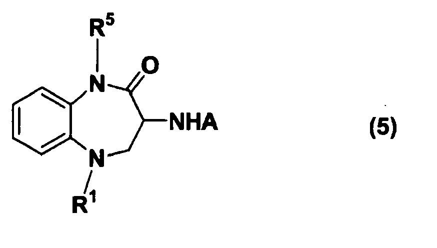 (wherein R¹ represents a linear, branched, or cyclic alkyl group; R⁵ represents a hydrogen atom, an alkyl group, or an acylalkyl group; and A represents a protective group containing a sulfonyl group or a carbonyl group),
the method comprising reacting an aniline derivative represented by formula (1): 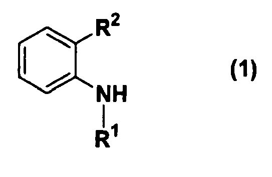 (wherein R² represents a group producing an amino group, an alkylamino group, or an acylalkylamino group through a reduction reaction or a hydrolysis reaction; and R¹ has the same meaning as defined above),
with an aziridine derivative represented by formula (2) : 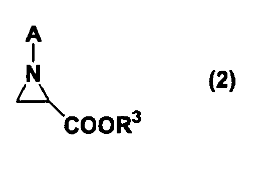 (wherein R³ represents an ester residue; and A has the same meaning as defined above),
to obtain a compound represented by formula (3): 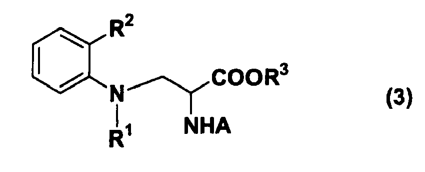 (wherein R¹, R², R³, and A respectively have the same meanings as defined above),
subjecting the compound thus obtained to a reduction reaction or a hydrolysis reaction to obtain a compound represented by formula (4): 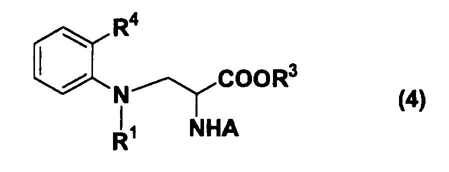 (wherein R⁴ represents an amino group, an alkylamino group, or an acylalkylamino group; and R¹, R³, and A respectively have the same meanings as defined above),
and then subjecting the compound thus obtained to a ring-closure reaction.

3. A method for producing a compound represented by formula (A) or a salt thereof: 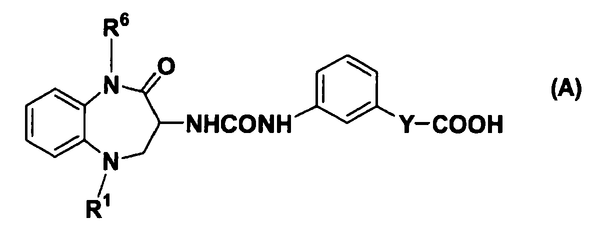 (wherein R¹ represents a linear, branched, or a cyclic alkyl group; R⁶ represents an alkyl group or an acylalkyl group; and Y represents a single bond or an alkylidene group),
the method comprising reacting an aniline derivative represented by formula (1): 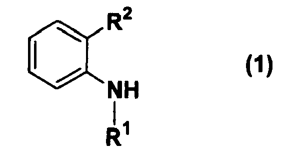 (wherein R² represents a group producing an amino group, an alkylamino group, or an acylalkylamino group through a reduction reaction or a hydrolysis reaction; and R¹ has the same meaning as defined above),
with an aziridine derivative represented by formula (2) : 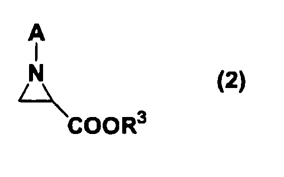 (wherein R³ represents an ester residue; and A represents a protective group containing a sulfonyl group or a carbonyl group),
to obtain a compound represented by formula (3): 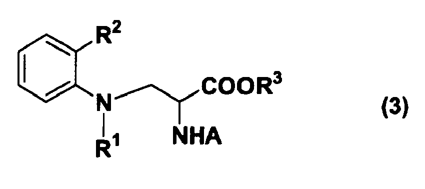 (wherein R¹, R², R³, and A respectively have the same meanings as defined above),
subjecting the compound thus obtained to a reduction reaction or a hydrolysis reaction to obtain a compound represented by formula (4): 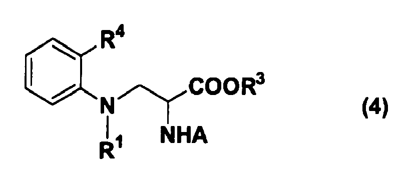 (wherein R⁴ represents an amino group, an alkylamino group, or an acylalkylamino group; and R¹, R³, and A respectively have the same meanings as defined above),
subsequently subjecting the compound thus obtained to a ring-closure reaction to obtain a 1,5-benzodiazepine derivative represented by formula (5): 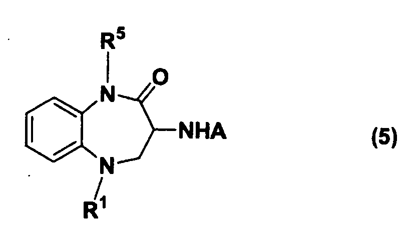 (wherein R⁵ represents a hydrogen atom, an alkyl group, or an acylalkyl group; and R¹ and A respectively have the same meanings as defined above),
allowing the 1,5-benzodiazepine derivative, when R⁵ is a hydrogen atom, to react with an alkyl halide or an acylalkyl halide, subsequently detaching the protective group A to obtain a compound represented by formula (6): 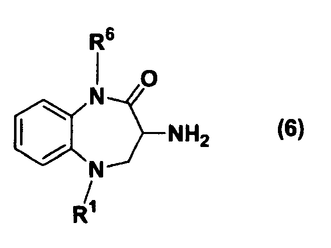 (wherein R¹ and R⁶ respectively have the same meanings as defined above), and
(a) reacting the compound (6) with a compound represented by formula (7): 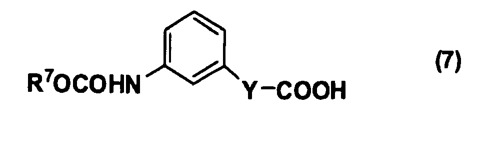 (wherein R⁷ represents an aryl group which may be substituted; and Y has the same meaning as defined above), or
(b) reacting the compound (6) with a halogenoformic acid aryl ester, and then reacting the resulting product with a compound represented by formula (8): 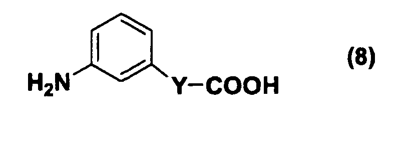 (wherein Y has the same meaning as defined above).

4. The method according to claim 3, wherein after the compound represented by formula (A) is obtained, the compound of formula (A) is converted to a calcium salt, and thereby a calcium salt of the compound of formula (A) is produced.

5. A compound represented by formula (3a) or a salt thereof: 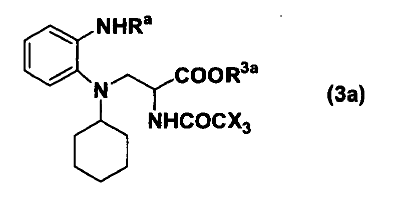 (wherein R^{a} represents a hydrogen atom or a benzyloxycarbonyl group; R^{3a} represents a hydrogen atom or a benzyl group; and X represents a halogen atom).

6. A compound represented by formula (5a) or a salt thereof: 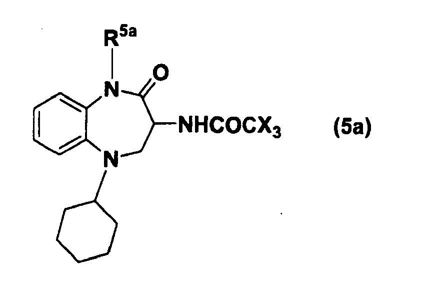 (wherein R^{5a} represents a hydrogen atom or a 3,3-dimethyl-2-oxobutylgroup; and X represents a halogen atom).

7. A compound represented by formula (1a) or a salt thereof: 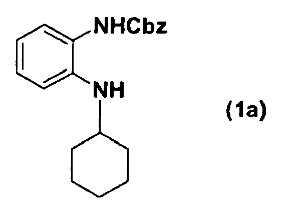 (wherein Cbz represents a benzyloxycarbonyl group).

8. A compound represented by formula (2a) or a salt thereof: 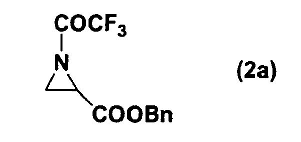 (wherein Bn represents a benzyl group).
